(19) Europäisches Patentamt / European Patent Office / Office européen des brevets

(11) EP 4 523 787 A1

(12) # EUROPEAN PATENT APPLICATION
published in accordance with Art. 153(4) EPC

(43) Date of publication:
**19.03.2025 Bulletin 2025/12**

(21) Application number: **23803831.9**

(22) Date of filing: **10.05.2023**

(51) International Patent Classification (IPC):
***B01J 23/02*** (2006.01) ***B01J 35/10*** (2006.01)
***B01J 37/00*** (2006.01) ***C07C 37/11*** (2006.01)
***C07C 39/06*** (2006.01) ***B01J 21/10*** (2006.01)

(52) Cooperative Patent Classification (CPC):
**B01J 21/10; B01J 23/02; B01J 35/60; B01J 37/00; C07C 37/11; C07C 39/06**

(86) International application number:
**PCT/KR2023/006330**

(87) International publication number:
**WO 2023/219408 (16.11.2023 Gazette 2023/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.05.2022 KR 20220058310**
**27.04.2023 KR 20230055405**

(71) Applicant: **Hanwha Solutions Corporation**
**Jung-gu**
**Seoul 04541 (KR)**

(72) Inventors:
- **LEE, Jong Kwon**
  **Daejeon 34128 (KR)**
- **CHOI, Min Suk**
  **Daejeon 34128 (KR)**
- **JEON, Bongsik**
  **Daejeon 34128 (KR)**
- **YUN, Seong Ho**
  **Daejeon 34128 (KR)**
- **CHUN, Jeong Hwan**
  **Daejeon 34128 (KR)**

(74) Representative: **Berggren Oy**
**P.O. Box 16**
**Eteläinen Rautatiekatu 10A**
**00101 Helsinki (FI)**

(54) **CATALYST FOR ORTHO-ALKYLATION REACTION, EXTRUSION MOLDING CATALYST FOR ORTHO-ALKYLATION REACTION, AND METHOD FOR PREPARING ORTHO-ALKYLATION REACTION PRODUCT USING SAME**

(57) The present disclosure relates to an ortho-alkylation reaction catalyst, an ortho-alkylation reaction extrusion molded catalyst, a preparation method of ortho-alkylation reaction product using the same, and more particularly, to an ortho-alkylation reaction catalyst and an ortho-alkylation reaction extrusion molded catalyst, which can minimize the material diffusion resistance of a catalyst in the ortho-alkylation reaction of a phenolic compound and thus can obtain an ortho-alkylation reaction product with high selectivity and conversion, and a preparation method of ortho-alkylation reaction product using the same.

*FIG. 1*

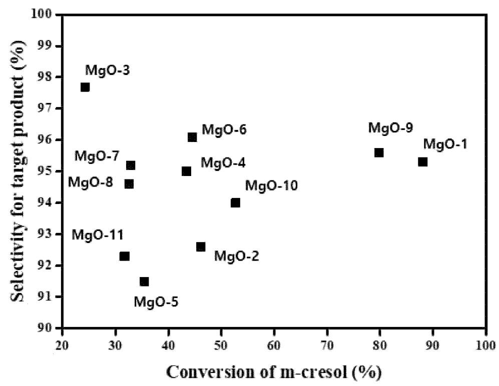


EP 4 523 787 A1

## Description

[TECHNICAL FIELD]

CROSS-REFERENCE TO RELATED APPLICATION(S)

This application claims priority to and the benefit of Korean Patent Application No. 10-2022-0058310, filed on May 12, 2022, and Korean Patent Application No. 10-2023-0055405, filed on April 27, 2023, with the Korean Intellectual Property Office, the disclosures of which are incorporated herein by reference in their entirety.

The present disclosure relates to an ortho-alkylation reaction catalyst, an ortho-alkylation reaction extrusion molded catalyst, a preparation method of ortho-alkylation reaction product using the same, and more particularly, to an ortho-alkylation reaction catalyst and an ortho-alkylation reaction extrusion molded catalyst, which can minimize the material diffusion resistance of a catalyst in the ortho-alkylation reaction of a phenolic compound and thus can obtain an ortho-alkylation reaction product with high selectivity and conversion, and a preparation method of ortho-alkylation reaction product using the same.

[BACKGROUND]

Alkylated hydroxyaromatic compounds are used for a variety of applications and are usually prepared by the gas-phase reaction of a phenol with methanol. Also, compounds with various structures can be prepared through further alkylation reactions, which are easily applicable to high-performance thermoplastic product groups, etc.

Such further alkylation reactions were usually performed in the presence of magnesium compounds, and various studies have been conducted to optimize the performance of magnesium-based catalysts.

In the alkylation reaction, the magnesium-based catalysts are required to have high activity, long active life, and high selectivity for a desired reaction product. Most of the alkylation catalysts used in the past mass-produced para-alkylated products, but various studies have been conducted to obtain more useful ortho-alkylation products in high yields.

However, a conventional technology is a technology that further uses a cocatalyst compound in combination with a magnesium-based catalyst, changes the composition of the catalyst, or change the reaction conditions. The conventional technology has the problem that it is difficult to obtain ortho-alkylation products with high selectivity and yield at a desired degree.

Therefore, in the ortho-alkylation reaction, there is a need to develop catalysts improved in terms of catalyst selectivity, catalyst activity, production yield, cost reduction, and overall productivity.

Meanwhile, for the alkylation reaction, it is possible to use small amounts of powdered catalysts at a laboratory level, but in order to mass-produce catalysts and apply them to a commercially available fixed-bed reactor, the catalyst must be appropriately molded to adapt to the reactor, taking into account the pressure drop during the reaction.

Typically, the methods often used in the methods for molding catalysts are extrusion molding and tablet molding. Tablet molding can produce accurate molded body shapes, but the equipment is expensive and the production unit cost is high, which significantly reduces the economic efficiency. On the other hand, extrusion molding is a molding method that is widely used because the equipment is simple and the production unit cost is relatively low. However, since molding the catalyst affects the internal/external material diffusion resistance, which can cause a decrease in activity in the molded catalyst. Therefore, various studies are needed to minimize this decrease in activity.

[DETAILED DESCRIPTION OF THE INVENTION]

[Technical Problem]

It is an object of the present disclosure to provide an ortho-alkylation reaction catalyst and an ortho-alkylation reaction extrusion molded catalyst, which can achieve excellent conversion and yield in the preparation of a selective ortho-alkylation reaction product.

It is another object of the present disclosure to provide an ortho-alkylation reaction catalyst and an ortho-alkylation reaction extrusion molded catalyst, which can achieve uniform catalytic activity inside and outside a molded catalyst without a decrease in activity.

It is yet another object of the present disclosure to provide a preparation method of ortho-alkylation reaction product using the catalyst.

[Technical Solution]

In order to achieve the above objects, provided herein is an ortho-alkylation reaction catalyst comprising:

magnesium oxide having a bimodal pore structure and a BET surface area of 100 $m^2/g$ to 180 $m^2/g$.

Also provided herein is an ortho-alkylation reaction extrusion molded catalyst, comprising magnesium oxide, and

comprising a macro pore having a diameter of 50 nm to 10,000 nm and a meso pore having a diameter of 2 nm to 50 nm,
wherein a BET surface area is 45 $m^2/g$ to 180 $m^2/g$.

Further provided herein is a method for preparing the ortho-alkylation reaction catalyst.

Specifically, provided herein a method for preparing the ortho-alkylation reaction catalyst, comprising:

a step 1 of preparing a mixture of magnesium oxide having a bimodal pore structure and a BET surface area of 100 $m^2/g$ to 180 $m^2/g$, an organic binder and a solvent; and
a step 2 of extrusion-molding the mixture.

Further provided herein is an ortho-alkylation reaction composition comprising: the ortho-alkylation reaction catalyst or the ortho-alkylation reaction extrusion molded catalyst, and a monomer composition comprising a meta-alkyl substituted phenolic monomer.

Further provided herein is a preparation method of ortho-alkylation reaction product, comprising:
a step performing an alkylation reaction of a monomer composition containing a meta-alkyl substituted phenolic monomer in the presence of the ortho-alkylation reaction catalyst or the ortho-alkylation reaction extrusion molded catalyst.

[Advantageous Effects]

An ortho-alkylation reaction catalyst according to the present disclosure exhibits high catalytic activity by using magnesium oxide having specific physical properties and pore structure, and when an ortho-alkylation reaction product is prepared using the catalyst, it exhibits remarkably high selectivity and conversion.

An ortho-alkylation reaction catalyst according to the present disclosure exhibits high catalytic activity by itself without the use of a separate cocatalyst or additional additives, and when an ortho-alkylation reaction product is prepared using the catalyst, it exhibits remarkably high selectivity and conversion.

An ortho-alkylation reaction extrusion molded catalyst according to the present disclosure exhibits high catalytic activity, and when an ortho-alkylation reaction product is prepared using the catalyst, it exhibits remarkably high selectivity and conversion.

In addition, an ortho-alkylation reaction extrusion molded catalyst according to the present disclosure includes the macro pore and meso pore appropriately formed, and has an appropriate BET specific surface area, and therefore provides an ortho-alkylation reaction extrusion molded catalyst that can minimize the material diffusion resistance of the catalyst and achieve uniform and excellent catalytic activity inside and outside the molded catalyst without reducing activity.

An ortho-alkylation reaction extrusion molded catalyst according to the present disclosure is prepared by using magnesium oxide having specific physical properties, an organic binder and a solvent in combination, and thus exhibits high catalytic activity by itself without the use of a separate cocatalyst or additional additives, and when an ortho-alkylation reaction product is prepared using the catalyst, it exhibits remarkably high selectivity and conversion.

[BRIEF DESCRIPTION OF THE DRAWINGS]

FIG. 1 is a graph showing the selectivity of the reaction product prepared using the ortho-alkylation reaction catalysts of Examples and Comparative Examples of the present disclosure.
FIG. 2 is an XDR graph of the ortho-alkylation reaction catalyst of Examples and Comparative Examples of the present disclosure.
FIG. 3 is a graph showing the pore distribution according to the $N_2$ adsorption-desorption analysis of the ortho-alkylation reaction catalysts of Examples and Comparative Examples of the present disclosure.
FIG. 4 is a graph showing the conversion versus selectivity according to the type of catalyst in the ortho-alkylation reaction extrusion molded catalyst of the Examples and Comparative Examples of the present disclosure.
FIG. 5 is a graph showing the conversion versus selectivity according to the type/content of the binder in the ortho-alkylation reaction extrusion molded catalysts of Examples of the present disclosure.
FIG. 6 is a graph showing the pore size versus pore volume according to the type of the molded catalysts of Examples and Comparative Examples of the present disclosure.

[DETAILED DESCRIPTION OF THE EMBODIMENTS]

Although the present disclosure may have various forms and various modifications may be made thereto, specific examples will be exemplified and explained in detail below. However, it is not intended to limit the present disclosure to specific disclosure, and it should be understood that the present disclosure includes all the modifications, equivalents or replacements thereof without departing from the spirit and technical scope of the present disclosure.

The technical terms used herein is only to explain exemplary embodiments and is not intended to limit the scope of the present disclosure. The singular forms "a," "an" and "the" are intended to include plural forms, unless the context clearly indicates otherwise. It should be understood that the terms "comprise," "include", "have", etc. are used herein to specify the presence of stated features, steps, components or combinations thereof, but do not preclude the presence or addition of one or more other features, steps, components, or combinations thereof.

Additionally, the word "on" or "above," as used in the context of formation or construction of one element, means pertaining to the direct formation or construction of one element on another element directly or the additional formation or construction of one element between layers or on a subject or substrate.

**I. Ortho-alkylation reaction catalyst**

(A. Ortho-alkylation reaction catalyst)

Magnesium-based catalysts usually used in the alkylation reaction are required to have high activity, long active life, and high selectivity for the desired reaction product. Most of the alkylation catalysts used in the past mass-produced para-alkylated products, but various studies have been conducted to obtain more useful ortho-alkylation products in high yields.

Specifically, recently, a variety of technologies have been developed to further use a cocatalyst compound in combination with a magnesium-based catalyst, change the composition of the catalyst, or change the reaction conditions. However, these technologies have the problem that it is difficult to obtain ortho-alkylation products having high selectivity and yield at a desired degree.

In addition, the present inventors have confirmed that in order to solve these problems, the BET specific surface area and pore structure of the magnesium oxide catalyst is set to a specific range, whereby significantly high catalytic activity can be realized in the alkylation reaction. The present inventors have found that an ortho-alkylation reaction catalyst according to the present disclosure exhibits excellent catalytic activity even by a single catalyst without further using a cocatalyst or adjusting the reaction conditions, and when an ortho-alkylation reaction product is prepared using the catalyst, it can exhibit remarkably high selectivity and conversion, and completed the present disclosure.

An ortho-alkylation reaction catalyst according to an embodiment of the disclosure comprises magnesium oxide having a bimodal pore structure and a BET surface area of 100 $m^2/g$ to 180 $m^2/g$.

Generally, the catalytic activity of a catalyst is determined by the reaction conditions or the acid-base characteristics of the catalyst. In the alkylation reaction of a meta-alkyl substituted phenolic monomer, if the catalyst has basic characteristics, it perpendicularly adsorbs reactants and favors ortho-C-alkylation (see Figure (a) below). Accordingly, the ortho-alkylation reaction catalyst according to the present disclosure contains a magnesium oxide (MgO) component.

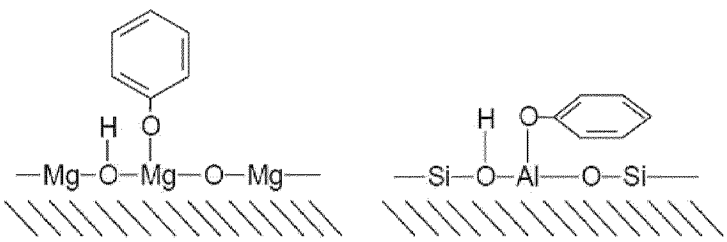

(a) Perpendicular Orientation Parallel Orientation

The magnesium oxide has a bi-modal pore structure, specifically, a mesopore bi-modal shape, and thus, facilitates the diffusion of the reactants, thereby enabling the reaction to performed effectively. In addition, by simultaneously satisfying the above-mentioned specific range of BET surface areas, it has excellent catalytic activity in the ortho-alkylation reaction, and can exhibit high selectivity, conversion, and yield.

Usually, as the BET surface area is larger, the active sites of the catalyst are increased, but in the case of a single-modal shape, even if the BET surface area is increased, the diffusion of the reactants is not smooth, which makes it difficult to perform the reaction to a desired degree.

Preferably, in the bi-modal pore structure, the diameter of the first pore may be 2 nm to 10 nm, and the diameter of

the second pore may be 10 nm to 50 nm. More preferably, the diameter of the first pore may be 4 nm to 8 nm, and the diameter of the second pore may be 20 nm to 45 nm or 35 nm to 45 nm. The bimodal pore structure having diameters in the above range makes it possible to exhibit the desired excellent catalytic activity, improved selectivity, conversion and yield.

The magnesium oxide satisfies the BET specific surface area of 100 $m^2/g$ to 180 $m^2/g$, and has many reaction active sites with a relatively large specific surface area, and thus is excellent in catalytic activity. At the same time, as mentioned above, the magnesium oxide has a bimodal pore structure and also facilitates the diffusion of reactants, thereby enabling the reaction to be performed effectively. As a result, the magnesium oxide has excellent catalytic activity in the ortho-alkylation reaction, and can exhibit improved selectivity, conversion and yield. When the BET specific surface area is less than 100 $m^2/g$, the reaction active sites are significantly reduced, and the conversion of the reactants is lowered, which makes it difficult to achieve a desired activity. In addition, when the BET specific surface area exceeds 180 $m^2/g$, the reaction active sites are increased, but the first pores are mainly formed, and the diffusion of reactants/products is not smooth, which makes it difficult to achieve high activity.

Preferably, the BET specific surface area of magnesium oxide may be 130 $m^2/g$ to 180 $m^2/g$, more preferably 130 $m^2/g$ to 150 $m^2/g$, and within the above range, it is possible to realize excellent catalytic activity without the above-mentioned problems, which is preferable.

The ortho-alkylation reaction catalyst may have a granule shape, and preferably, it may have a particle size of about 212 $\mu$m to 425 $\mu$m. Within the above particle size range, it is possible to realize excellent catalytic activity, which is preferable.

(B. Ortho-alkylation reaction extrusion molded catalyst)

Meanwhile, for the alkylation reaction, it is possible to use small amounts of powdered catalysts at the laboratory level, but in order to mass-produce catalysts and apply them to a commercially available fixed-bed reactor, the catalyst must be appropriately molded to adapt to the reactor, taking into account the pressure drop during the reaction. The most commonly used methods for molding catalysts are extrusion molding and tablet molding. Tablet molding can produce accurate molded body shapes, but the equipment is expensive and the production unit cost is high, which significantly reduces the economic efficiency. On the other hand, extrusion molding is a molding method that is widely used because the equipment is simple and the production unit cost is relatively low. However, since extrusion-molding the catalyst affects the internal/external material diffusion resistance, which can cause a problem that a decrease in activity occurs in the prepared extrusion molded catalyst.

Thus, to solve these problems, the present inventors have derived the main factors that affects the catalytic activity of a molded catalyst at the time of preparation by extrusion molding. Specifically, the inventors have confirmed that as mentioned above, magnesium oxide having a specific pore structure is used, and at the same time, macro-sized pores and meso-sized pores are appropriately molded in the final catalyst to realize an appropriate BET specific surface area, thereby minimizing the effect on internal/external material diffusion resistance and realizing uniform catalytic activity in the prepared extrusion molded catalyst, and completed the present disclosure.

In addition, the present inventors have confirmed that, for an extrusion molded catalyst prepared by including a specific magnesium oxide, when the catalyst satisfies an appropriate pore distribution and BET specific surface area, it is possible to achieve remarkably high catalytic activity in an alkylation reaction. In this regard, the inventors have found that the ortho-alkylation reaction catalyst according to the present disclosure exhibits excellent catalytic activity even by a single catalyst without further using a cocatalyst or adjusting reaction conditions, and when an ortho-alkylation reaction product is prepared using the catalyst, it can exhibit remarkably high selectivity and conversion, and completed the present disclosure.

An ortho-alkylation reaction extrusion molded catalyst according to an embodiment of the present disclosure comprises magnesium oxide, and comprises a macro pore having a diameter of 50 nm to 10,000 nm and a meso pore having a diameter of 2 nm to 50 nm, wherein a BET surface area satisfies 45 $m^2/g$ to 180 $m^2/g$.

As mentioned above, the catalytic activity of a catalyst is generally determined by the reaction conditions or the acid-base characteristics of the catalyst. As a result, the ortho-alkylation reaction extrusion molded catalyst according to the present disclosure includes a magnesium oxide(MgO) component, so that the catalyst has basic characteristics, perpendicularly adsorbs the reactant, and favors ortho-C-alkylation (see Figure (a) above).

The catalyst is prepared by using magnesium oxide having a bi-modal pore structure, wherein the magnesium oxide used in the preparation step specifically has a bimodal shape of a meso-sized pore (mesopore), thereby facilitating the diffusion of reactants and enabling the reaction to be performed effectively. As a result, the magnesium oxide has excellent catalytic activity in the ortho-alkylation reaction and can exhibit high selectivity, conversion and yield.

The ortho-alkylation reaction extrusion molded catalyst includes a macro pore having a diameter of 50 nm to 10,000 nm and a meso pore having a diameter of 2 nm to 50 nm.

In this way, the macro pore and the meso pore are appropriately formed to minimize the material diffusion resistance of the catalyst, which makes it possible to achieve uniform excellent catalytic activity inside and outside the

molded catalyst without a decrease in activity.

The macro pore of the ortho-alkylation reaction extrusion molded catalyst may be included in an amount of 1 to 10 vol%, preferably, 1 to 9 vol%, 2 to 10 vol%, 2 to 9 vol%, or 2 to 5 vol% based on the total mixed volume of the macro pore and the meso pore. When included in the above range, the material diffusion resistance of the molded catalyst can be minimized, which is thus preferable.

The meso pore of the ortho-alkylation reaction extrusion molded catalyst may be included in an amount of 90 to 99 vol%, preferably, 90 to 98 vol%, 91 to 98 vol%, or 95 to 98 vol%, based on the total mixed volume of the macro pore and the meso pore. When included in the above range, the molded catalyst is suitable for realizing excellent catalytic activity without a decrease in activity.

The method for measuring the pore size and the pore volume distribution of the catalyst will be described in more detail in Experimental Examples described hereinafter.

The ortho-alkylation reaction extrusion molded catalyst has a BET specific surface area of 45 $m^2/g$ to 180 $m^2/g$.

The catalyst has the macro pore and meso pore appropriately formed and at the same time exhibits a specific range of BET specific surface area values, and thus can minimize the material diffusion resistance of the catalyst, thereby achieving uniform and excellent catalytic activity inside and outside the molded catalyst without a decrease in activity.

Preferably, the BET specific surface area may be 45 $m^2/g$ to 150 $m^2/g$, 45 $m^2/g$ to 130 $m^2/g$, or 60 $m^2/g$ to 130 $m^2/g$, and when a specific surface area in the above range is realized, the material diffusion resistance of the molded catalyst can be minimized, which is preferable. The method for measuring the BET specific surface area of the catalyst will be described in more detail in Experimental Examples described hereinafter.

Meanwhile, the pore distribution and the BET specific surface area of the catalyst can be preferably achieved by combining an organic binder and a solvent together with magnesium oxide having specific physical properties. This will be described in more detail in the section of the preparation method.

The ortho-alkylation reaction extrusion molded catalyst can satisfy a size of 0.5 mm to 6.0 mm, and can realize uniform and excellent catalytic activity within the range. More preferably, the size of the catalyst may be 1.0 mm to 3.0 mm or 1.2 to 2.0 mm. If the size of the catalyst is too small outside the above range, a large pressure drop may occur during the alkylation reaction, which may be problematic, and if the catalyst size is too large outside the above range, there may a concern that the catalytic activity is decreased.

The size of the catalyst can be controlled according to the diameter of the extrusion die used in the final extrusion step in the preparation process.

Usually, the shape of the extrusion molded catalyst is realized into a cylindrical shape by the extrusion die, wherein the extrusion molded catalyst according to the present disclosure may have a ratio of 1:1 ($\pm$0.1) between the diameter of the circular cross-section of the cylinder and the length of the cylinder.

Therefore, the size of the extrusion molded catalyst may mean the diameter of the circular cross section of the cylinder and/or the length of the cylinder, and it is preferable that both the circular cross section and the length of the cylinder simultaneously satisfy the above range.

The specific method of measuring the size of the catalyst will be described in more detail in Experimental Examples described hereinafter.

## II. Method for preparing an ortho-alkylation reaction extrusion molded catalyst

According to yet another embodiment of the disclosure, there is provided a method for preparing an ortho-alkylation reaction extrusion molded catalyst comprising the following steps is provided as the above-mentioned method for preparing the ortho-alkylation reaction extrusion molded catalyst (B).

Specifically, the method comprises a step 1 of preparing a mixture of magnesium oxide having a bimodal pore structure and a BET surface area of 100 $m^2/g$ to 180 $m^2/g$, an organic binder and a solvent; and a step 2 of extrusion-molding the mixture.

Hereinafter, each step will be described in detail.

### Step 1: Step of preparing the mixture

First, a mixture of magnesium oxide having a bimodal pore structure and a BET surface area of 100 $m^2/g$ to 180 $m^2/g$, an organic binder and a solvent is prepared.

The magnesium oxide can be applied in the same manner as described for the ortho-alkylation reaction extrusion molded catalyst.

Preferably, the magnesium oxide has a bi-modal pore structure, specifically, a bi-modal shape in the meso-sized pore (mesopore) range, thereby facilitating the diffusion of the reactants and enabling the reaction to be performed effectively. This makes it possible to have excellent catalytic activity in the ortho-alkylation reaction and exhibit high selectivity, conversion and yield.

Typically, as the BET surface area is larger, the active sites of the catalyst are increased, but in the case of a single-modal shape, even if the BET surface area is increased, the diffusion of the reactants is not smooth, which makes it difficult to perform the reaction to a desired degree.

Preferably, in the bi-modal pore structure, the diameter of the first pore may be 2 nm to 10 nm, and the diameter of the second pore may be 10 nm to 50 nm. More preferably, the diameter of the first pore may be 4 nm to 8 nm, and the diameter of the second pore may be 20 nm to 45 nm or 35 nm to 45 nm. The bi-modal pore structure having the diameter in the above range makes it possible to exhibit the desired excellent catalytic activity, improved selectivity, conversion and yield.

The magnesium oxide satisfies the BET specific surface area of 100 $m^2/g$ to 180 $m^2/g$, and has many reaction active sites with a relatively large specific surface area, and thus is excellent in catalytic activity. At the same time, as mentioned above, the magnesium oxide has a bimodal pore structure and also facilitates the diffusion of reactants, thereby enabling the reaction to be performed effectively. As a result, the magnesium oxide has excellent catalytic activity in the ortho-alkylation reaction, and can exhibit improved selectivity, conversion and yield. When the BET specific surface area is less than 100 $m^2/g$, the reaction active sites are significantly reduced, and the conversion of the reactants is lowered, which makes it difficult to achieve a desired activity. In addition, when the BET specific surface area exceeds 180 $m^2/g$, the reaction active sites are increased, but the first pores are mainly formed, and the diffusion of reactants/products is not smooth, which makes it difficult to achieve high activity.

Preferably, the BET specific surface area of magnesium oxide may be 130 $m^2/g$ to 180 $m^2/g$, more preferably 130 $m^2/g$ to 150 $m^2/g$, and within the above range, it is possible to realize excellent catalytic activity without the above-mentioned problems, which is preferable.

The organic binder is a component that has important effects on uniformly binding magnesium oxide during the preparation of a molded catalyst to make it easier to prepare the catalyst, and particularly, forming appropriate pores inside the catalyst through baking during the preparation of an extrusion molded catalyst, thereby realizing the uniformity of the catalyst activity. When magnesium oxides having the above-mentioned specific physical properties are used alone, the ease of catalyst preparation may be reduced, and the formation of the desired pores may be slightly difficult, but if the magnesium oxide is used in combination, a more excellent catalyst activity can be uniformly achieved, which is preferable.

Specific examples of the organic binder include methyl cellulose, carboxymethyl cellulose, ethylene glycol, polyethylene glycol, polyphenylene oxide, glycerin and propylene glycol, which may be used alone or in combination of two or more. More preferably, methylcellulose, carboxymethylcellulose, polyethylene glycol (PEG 20000 or PEG 400) can be used.

The organic binder may be included in an amount of 0.1 to 20 parts by weight, preferably, 0.1 to 15 parts by weight, 0.1 to 10 parts by weight, or 1 to 5 parts by weight, based on 100 parts by weight of magnesium oxide. The use of an organic binder in the above range is suitable for achieving the above-mentioned effects.

The solvent is used so that the mixture can be kneaded evenly, and makes it possible to easily perform extrusion when producing an extrusion molded body.

Specific examples of the solvent include water, alcohol, etc., which may be used alone or in combination of two or more. More preferably, water may be used. The water may be distilled water, ion-exchanged water, or high-purity water such as deionized water(DIW). When the water contains impurities, the impurities may adhere to the catalyst and reduce the activity of the catalyst, so it is preferable to use ultrapure water or the like. In the case of alcohol, a primary alcohol of $C_3$ or higher may be used.

The solvent may be included in an amount of 50 to 200 parts by weight, preferably, 80 to 150 parts by weight or 100 to 120 parts by weight, based on 100 parts by weight of magnesium oxide. The mixture mixed in the above ratio may preferably be in the form of a paste In this case, the extrusion molding process may be easily performed, which is preferable.

Meanwhile, according to an embodiment of the present disclosure, additional additives may be optionally used in the mixture, and for example, a lubricant may be used so that the final molded catalyst can be prepared uniformly or without crushing during the preparation process. Specific examples of the lubricant include, but are not limited to, magnesium stearate, aluminum stearate, graphite, and the like.

In addition, other additives used in the art can be used without special restrictions within the range that does not impair the desired physical properties.

Step 2: Extrusion molding step

Next, the method comprises a step 2 of extrusion-molding the mixture.

Typically, tablet molding, which is used in a catalyst molding method, can produce accurate molded body shapes, but the equipment is expensive, the production unit cost is high, and the economic efficiency is significantly reduced. On the other hand, extrusion molding is a molding method that is widely used because the equipment is simple and the production unit cost is relatively low. However, when the catalyst is molded, it may affect the internal/external material

diffusion resistance, which may cause a decrease in activity in the molded catalyst. However, in the present disclosure, magnesium oxide having specific physical properties, an organic binder, and a solvent are used in combination as described above, so that it is possible to prepare an extrusion molded catalyst that has high activity without such problems, and at the same time has uniformity inside and outside the catalyst.

The extrusion molding process can generally be performed by using a screw type extruder and a piston type extruder, but preferably, it can be performed by using a single piston extrusion molding machine, and in this case, it is possible to prepare an extrusion molded catalyst having a low moisture content and high compression ratio at high pressure. In this case, the extrusion process may be performed through a die of a specific diameter, but is not limited thereto.

The average diameter of the extrusion die used may be appropriately adjusted in accordance with a desired diameter range of the final molded catalyst.

Additional step: drying and baking step

In an embodiment of the present disclosure, the method may further comprise drying and baking steps after the extrusion molding.

The process conditions for5 the drying and baking steps may be applied without any particular limitation to processes commonly applied in the art.

Specifically, the temperature for performing the drying step is not particularly limited, but the drying may be, for example, 80°C to 120°C, preferably 90°C to 110°C.

The time for performing the drying step is not particularly limited, but may be performed for, for example, 1 hour to 13 hours, preferably 3 hours to 8 hours, or 4 hours to 6 hours.

The temperature for performing the baking step is not particularly limited, but the baking may be performed at, for example, 300°C to 600°C, and preferably 400°C to 550°C.

The time for performing the baking step is not particularly limited, but is performed for an appropriate amount of time or longer in order to remove the organic binder and solvent in the mixture and form the desired pores in the catalyst. Preferably, the baking can be performed for 1 to 9 hours, or 3 to 6 hours.

The catalyst produced by the above-mentioned preparation method of an ortho-alkylation reaction extrusion molded catalyst uses magnesium oxide having specific physical properties, an organic binder and a solvent in combination, thereby achieving uniform and excellent catalytic activity inside and outside the molded catalyst without a decrease in activity.

**III. Ortho-alkylation reaction composition**

According to an embodiment of the present disclosure, the ortho-alkylation reaction composition comprises: the above-mentioned ortho-alkylation reaction catalyst (A) or the ortho-alkylation reaction extrusion molded catalyst (B), and a monomer composition comprising a meta-alkyl substituted phenolic monomer.

The ortho-alkylation reaction catalyst (A) includes magnesium oxide having a bimodal pore structure and a BET surface area of 100 $m^2/g$ to 180 $m^2/g$. In this regard, the above-mentioned contents are all applicable to this catalyst. Specifically, the magnesium oxide has a bimodal pore structure, and specifically has a bi-modal shape of mesopores, so that the diffusion of the reactants is made smooth, thereby enabling the reaction to be performed effectively. In addition, by simultaneously satisfying the above-mentioned specific range of BET surface areas, it has excellent catalytic activity in the ortho-alkylation reaction and can exhibit high selectivity and conversion.

In addition, the ortho-alkylation reaction extrusion molded catalyst (B) contains magnesium oxide, includes a macro pore having a diameter of 50 nm to 10,000 nm and a meso pore having a diameter of 2 nm to 50 nm, and has a BET surface area of 45 $m^2/g$ to 180 $m^2/g$. In this regard, the above-mentioned contents are all applicable to this catalyst. Specifically, the extrusion molded catalyst has the above-mentioned macro pore and meso pore appropriately formed to minimize the material diffusion resistance of the catalyst, so that uniform and excellent catalytic activity can be achieved inside and outside the molded catalyst without a decrease in activity.

In addition, the magnesium oxide included in the ortho-alkylation reaction extrusion molded catalyst has a bi-modal pore structure, and specifically, it has a bi-modal shape of mesopores, which facilitates the diffusion of the reactants, thereby enabling the reaction to be performed effectively. Furthermore, by simultaneously satisfying the above-mentioned specific range of BET surface areas, it is possible to have excellent catalytic activity in the ortho-alkylation reaction and exhibit high selectivity and conversion.

In the monomer composition, the meta-alkyl substituted phenolic monomer may be meta-cresol.

In addition to the meta-alkyl-substituted phenol monomer, the monomer composition further comprise an alkanol and distilled water (DI Water), wherein an alkyl group can be introduced through a reaction with the alkanol, and the distilled water is used together, thereby suppressing the decomposition reaction of the alkanol, which is preferable. The alkanol

can preferably be methanol.

The monomer composition may preferably contain phenolic monomer: alkanol: distilled water in a ratio of 1:3 to 10:1 to 5 parts by weight, more preferably in a ratio of 1:4 to 6:1 to 3 parts by weight. In this case, if the alkanol is contained in a small amount outside the above range, the alkylation mediator (agent) will decrease, and the conversion becomes lower. If distilled water is contained in a small amount outside the above range, the effect of suppressing the decomposition reaction of alkanol is reduced, and if the alkanol or distilled water is contained in an excessive amount outside the above range, the phenolic monomer may be competitively adsorbed to the catalyst active site, which results in a problem of reduced reactivity.

When the content is within the above range, it is preferable that an ortho-alkylation reaction product can be prepared with the desired selectivity and conversion.

**IV. Method for producing ortho-alkylation reaction product**

According to an embodiment of the present disclosure, a preparation method of ortho-alkylation reaction product comprises: performing an alkylation reaction of a monomer composition containing a meta-alkyl substituted phenolic monomer in the presence of the above-mentioned ortho-alkylation reaction catalyst (A) or the ortho-alkylation reaction extrusion molded catalyst (B). Specifically, the method for preparing an ortho-alkylation reaction product can be performed using an ortho-alkylation reaction composition comprising the above-mentioned ortho-alkylation reaction catalyst or the ortho-alkylation reaction extrusion molded catalyst. The details of the catalyst and the reaction composition can be applied similarly to all of the contents set forth above.

Specifically, by including the above-mentioned ortho-alkylation reaction catalyst (A) or the ortho-alkylation reaction extrusion molded catalyst B, it is possible realize uniform and excellent catalytic activity inside and outside the molded catalyst without a decrease in activity.

In the method for preparing the ortho-alkylation reaction product, the monomer composition may be applied similarly to all the contents set forth above. Specifically, the monomer composition may be a meta-alkyl substituted phenolic monomer, such as meta-cresol. The method for preparing the ortho-alkylation reaction product may exhibit high selectivity and conversion of the desired ortho-alkylation reaction product by using a reaction composition comprising the above-mentioned extrusion molded catalyst.

The ortho-alkylation reaction product may be, for example, at least one selected from the group consisting of 2,5-dimethylphenol, 2,3-dimethylphenol, 2,3,6-trimethylphenol, 3-methylanisole, 3,4-dimethylphenol, and tetramethylphenol, and may be prepared by selectively substituting an alkyl group at the ortho position in meta-cresol through a multi-step reaction as follows.

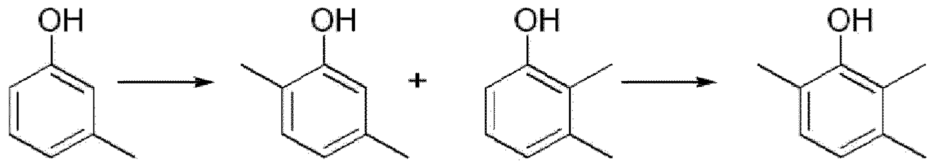

m-cresol 2,5-xylenol 2,3-xylenol 2,3,6-TMP

The alkylation reaction may be performed preferably at 350 to 550°C, more preferably at 350°C to 550°C. In this case, if the temperature is less than 350°C, the alkylation reaction may not be sufficiently activated, and if the temperature exceeds 550°C, a large amount of by-products are generated due to overreaction.

The alkylation reaction can be performed under inert conditions, for example, in the presence of an inert carrier gas. As the inert carrier gas, nitrogen, helium, neon, argon, etc. can be used, and preferably, nitrogen can be used.

The nitrogen flow rate is not particularly limited, but preferably, it is 5 to 70 cc/min, 10 to 50 cc/min, or 15 to 40 cc/min. By performing the reaction under the above conditions, it is possible to prepare an ortho-alkylation reaction product with the desired selectivity and conversion, which is preferable.

Meanwhile, before the introduction of the monomer composition in the alkylation reaction, a step of raising and maintaining the inside of the reactor to the activation temperature for catalyst activation can be performed, and the temperature range can be the same as the reaction temperature. The time for performing the catalyst activation is not particularly limited, but can be about 30 minutes to 1 hour.

The alkylation reaction can be preferably performed by using a continuous flow gas-phase reactor. In this case, the reaction can be performed while the monomer composition is injected into the continuous flow gas-phase reactor at a liquid hourly space velocity (LHSV) of 0.5 $hr^{-1}$ to 2.0 $hr^{-1}$. At this time, in the case of deviating the above space velocity rang, the reactant may not be sufficiently activated at the catalytic active site, which may make it difficult to obtain the product

smoothly, and the activity or selectivity of the catalyst may decrease, thereby reducing the amount of the product.

By performing the reaction under the above conditions, the ortho-alkylation reaction product can be prepared with the desired selectivity and conversion, which is preferable.

Hereinafter, the operation and effect of the present disclosure will be described in more detail by way of specific examples. However, these examples are presented for illustrative purposes only, and the scope of the present disclosure is not defined thereby.

**[Example 1 and Comparative Example 1: Ortho-alkylation reaction using ortho- alkylation reaction catalyst (A)]**

**Example 1-1**

The alkylation reaction of m-cresol was performed by using a continuous flow gas-phase reactor. First, magnesium oxide component (MgO-1, crystalline structure: MgO) having the physical properties shown in Table 1 ware prepared in a granule shape (212 - 425 um), and 1 g of the catalyst was charged into a stainless steel tube type reactor with a diameter of 1/2 inch x a length of 50 cm, and then installed in the main furnace.

Then, $N_2$, i.e., a carrier gas, was flowed (37.5 cc/min) using a mass flow controller (MFC), and the inside of the reactor was raised to the activation temperature (450°C) for catalyst activation and maintained for 1 hour. Then, the monomer composition (12.5 mg/min, m-cresol: methanol: D.I. water = 1:5:1, LHSV 0.75/h) was introduced through a pre-heater (250°C) and vaporized. A reaction composition including a catalyst and a monomer composition was introduced into the reactor, and then, an alkylation reaction (450°C, maintained at normal pressure) was performed for 5 hours to obtain a reaction product.

The product during the reaction was collected in a liquid form (IPA(isopropyl alcohol) was used as a solvent) for analysis.

**Example 1-2 and Comparative Examples 1-1 to 1-9**

The alkylation reaction was performed in the same manner as in Example 1-1, except that the magnesium oxide component was changed as shown in Table 1 below, to obtain a reaction product.

**[Experimental Example 1-1: Analysis of physical properties of ortho-alkylation reaction catalyst (A)]**

(1) XRD Analysis

The crystallite structure of the catalyst used in Examples and Comparative Examples was confirmed through XRD analysis, and the crystallite size was calculated through Scherrer Equation using the maximum peak value of the XRD patterns. The results are shown in Table 1 and FIG. 2.

In this case, K = 0.89 (the shape factor of the average crystallite), L = 1.5418 (the wavelength for Cu K$\alpha$), and FWHM represents the full width half maximum of the peak, and $\theta$ = the maximum peak position.

(2) $N_2$ adsorption-desorption analysis

The catalysts used in Examples and Comparative Examples were analyzed by $N_2$ adsorption-desorption analysis to confirm the BET(Brunauer-Emmett-Teller) surface area, pore volume, and size distribution.

The BET surface area was calculated using the adsorption value of P/P0 = 0.05-0.3, and the pore volume and size distribution were calculated using the desorption value, and the results are shown in Table 1 and FIG. 3.

[Table 1]

| Category | Type | crystallite size, nm | BET surface area, $m^2/g$ | Total Pore Volume, $cm^3/g$ | Pore Size, nm |
|---|---|---|---|---|---|
| Example 1-1 | Mg0-1 | 9 | 144.4 | 0.72 | 6, 40 |
| Example 1-2 | Mg0-9 | 10 | 134.2 | 0.7 | 6, 40 |
| Comparative Example 1-1 | Mg0-2 | 17 | 44.3 | 0.4 | 50 |
| Comparative Example 1-2 | Mg0-3 | 37 | 32.6 | 0.39 | 60 |
| Comparative Example 1-3 | Mg0-4 | 10 | 125.9 | 0.46 | 10 |

(continued)

| Category | Type | crystallite size, nm | BET surface area, $m^2/g$ | Total Pore Volume, $cm^3/g$ | Pore Size, nm |
|---|---|---|---|---|---|
| Comparative Example 1-4 | Mg0-5 | 12 | 107.7 | 0.29 | 7 |
| Comparative Example 1-5 | Mg0-6 | 40 | 25.7 | 0.2 | 25 |
| Comparative Example 1-6 | Mg0-7 | 5.4 | 195.4 | 0.77 | 4, 6 |
| Comparative Example 1-7 | Mg0-8 | 5.4 | 188.3 | 0.82 | 4, 6 |
| Comparative Example 1-8 | Mg0-10 | 13 | 102.7 | 0.72 | 8, 40 |
| Comparative Example 1-9 | Mg0-11 | 20 | 34.1 | 0.41 | 60 |

**[Experimental Example 1-2: Analysis of ortho-alkylation reaction products]**

The liquids in which the reaction products prepared in Example 1 and Comparative Example 1 were collected were analyzed by using a gas chromatograph(GC) device equipped with a flame ionization detector(FID). From the GC result values, the conversion of m-cresol and the selectivity for target products (2,3,6-TMP, 2,5-DMP, 2,3-DMP) were calculated using the following Mathematical Equations 1 and 2, and the results are shown in Table 2 below.

The target products were defined as the final product, 2,3,6-TMP, and intermediate materials, 2,5-DMP and 2,3-DMP, from which the final products can be obtained through further reaction.

[Mathematical Equation 1]

$$\text{Conversion of m-cresol (\%)} = \frac{\text{Mole number of reacted m-cresol}}{\text{Mole number of fed m-cresol}} \times 100$$

[Mathematical Equation 2]

$$\text{Selectivity for trarget product (\%)} = \frac{\text{Mole number of produced 2,5-DMP + 2,3-DMP + 2,3,6-TMP}}{\text{Mole number of reacted c-cresol}} \times 100$$

[Table 2]

| Category | Catalyst | Conversion of m-cresol | Selectivity (mol%) | | | | | | | |
|---|---|---|---|---|---|---|---|---|---|---|
| | | | Target product | A | B | C | D | E | F | G |
| Example 1-1 | Mg0-1 | 88.1 | 95.3 | 49.7 | 12.7 | 32.9 | 0.6 | 0.4 | 0.2 | 1.3 |
| Example 1-2 | Mg0-9 | 79.8 | 95.6 | 50.3 | 13.6 | 31.7 | 0.6 | 0.6 | 0.1 | 1.4 |
| Comparative Example 1-1 | Mg0-2 | 46.1 | 92.6 | 62.8 | 18.7 | 11.0 | 1.8 | 2.3 | 1.2 | - |
| Comparative Example 1-2 | Mg0-3 | 24.2 | 97.7 | 68.7 | 24.7 | 4.4 | 0.2 | 0.7 | 1.0 | - |
| Comparative Example 1-3 | Mg0-4 | 43.4 | 95.0 | 65.4 | 20.6 | 8.9 | 0.8 | 2.2 | 0.5 | 0.1 |
| Comparative Example 1-4 | Mg0-5 | 35.5 | 91.5 | 63.5 | 20.5 | 7.4 | 0.6 | 4.5 | 2.9 | 0.5 |
| Comparative Example 1-5 | Mg0-6 | 44.5 | 96.1 | 66.8 | 20.1 | 9.2 | 0.7 | 1.4 | 0.8 | - |
| Comparative Example 1-6 | Mg0-7 | 32.9 | 95.2 | 66.7 | 22.3 | 6.1 | 1.6 | 2.5 | - | - |
| Comparative Example 1-7 | Mg0-8 | 32.5 | 94.6 | 66.4 | 21.8 | 6.4 | 1.5 | 2.7 | 0.3 | 0.1 |
| Comparative Example 1-8 | Mg0-10 | 52.7 | 94.0 | 63.9 | 19.4 | 10.7 | 1.3 | 1.9 | 0.4 | 0.4 |

(continued)

| Category | Catalyst | Conversion of m-cresol | Selectivity (mol%) | | | | | | | |
|---|---|---|---|---|---|---|---|---|---|---|
| | | | Target product | A | B | C | D | E | F | G |
| Comparative Example 1-9 | Mg0-11 | 31.7 | 92.3 | 65.0 | 20.7 | 6.6 | 1.6 | 3.4 | 0.8 | 0.4 |

As can be seen from the experimental data in Table 2, it was confirmed that the catalyst including magnesium oxide having specific physical properties and pore structure of the present disclosure exhibits high catalytic activity even by a single catalyst, and when an ortho-alkylation reaction product is prepared using the catalyst, it can exhibit remarkably high selectivity and conversion.

**[Example 2 and Comparative Example 2: Ortho-alkylation reaction extrusion molded catalyst (B)]**

Example 2-1. Preparation of extrudate catalyst (Preparation of extrusion molded catalyst)

50 g of magnesium oxide (MgO powder, Crystallite size 9 nm, BET surface area: 144.4 $m^2/g$, Total pore volume: 0.72 $cm^3/g$, Pore size: 6, 40 nm), 1 g of organic binder (methyl cellulose), and 60 g of distilled water were placed in a mixing bowl and mixed evenly to prepare a paste. When the paste was completed, it was extruded through a 2 mm diameter die using a single piston extruder catalyst molding device. The noodle shapes extruded from the single piston extruder were evenly spread on a tray.

Next, it was dried at 100°C for 4 hours using a convection oven, and then the dried extrusion molded catalyst was cut into 2 mm intervals and baked at 450°C for 6 hours using a muffle furnace to prepare a magnesium-based extrusion molded catalyst (A-1).

Comparative Example 2-1. Preparation of tablet catalyst

Magnesium oxide (MgO powder, Crystallite size: 9 nm, BET surface area: 144.4 $m^2/g$, Total pore volume: 0.72 $cm^3/g$, Pore size: 6, 40 nm) were first tableted into a disc shape using a plate-type press equipment and an aluminum dish, and then pulverized to prepare granules with an appropriate particle size and provide flowability. 10 wt% of organic binder (PPO, polyphenylene oxide) and 1 wt% of a lubricant (magnesium stearate) were mixed into the prepared granules, and tablets with a diameter of 2.4 mm were produced using a single rotary tablet press catalyst molding equipment.

Next, it was dried at 100°C for 4 hours in a convection oven, and then the dried tablet-molded catalyst was baked at 450°C for 1 hour in a muffle furnace to complete a magnesium-based tablet-molded catalyst (B-1).

Examples 2-2 to 2-8

The same procedure was proceeded in the same manner as in Example 1-1, except that the components and contents of the binder resin and solvent used in Example 1-1 were changed as shown in Table 3 below.

[Table 3]

| Category | Type | Binder resin | Solvent |
|---|---|---|---|
| Example 2-1(A-1) | Extrudate | MC 1g | D.I. water 60g |
| Example 2-2(A-2) | Extrudate | MC 1g | D.I. water 60g |
| Example 2-3(A-3) | Extrudate | MC 0.2g | D.I. water 60g |
| Example 2-4(A-4) | Extrudate | MC 2g | D.I. water 60g |
| Example 2-5(A-5) | Extrudate | MC 10g | D.I. water 60g |
| Example 2-6(A-6) | Extrudate | MC 1g | D.I. water 72g |
| Example 2-7(A-7) | Extrudate | PEG 20000 1g | D.I. water 60g |
| Example 2-8(A-8) | Extrudate | PEG 400 1g | D.I. water 60g |
| Comparative Example 2-1(B-1) | Tablet | PPO 10wt%, Mg stearate 1wt% | - |
| <Catalyst Type><br>Extrudate: Extrusion molded catalyst | | | |

(continued)

| Category | Type | Binder resin | Solvent |
|---|---|---|---|
| Tablet: Tablet molded catalyst<br>< Binder resin><br>MC: Methyl cellulose<br>PEG: Polyethylene glycol<br>PPO: Polyphenylene oxide | | | |

**[Experimental Example 2-1: Analysis of ortho-alkylation reaction extrusion molded catalyst (B)]**

(1) Analysis of BET specific surface area

The catalysts of Example 1 and Comparative Example 1 were subjected to nitrogen adsorption/desorption analysis experiments, and the isothermal adsorption curve and the amount of adsorbed nitrogen at standard temperature and pressure were measured according to the BET (Brunauer Emmett Teller) calculation equation to determine the BET specific surface area value, and the results are shown in Table 4.

The average diameter of the catalyst in the form of an extrudate refers to the diameter of the circular cross section of a cylindrical catalyst prepared by a die mounted on a single piston extruder, and the average diameter of the catalyst in the form of a tablet refers to the diameter of the circular cross section of a cylindrical catalyst prepared by a punch mounted on a rotary tablet press.

[Table 4]

| Category | Size (average diameter)/ shape | BET specific surface area ($m^2$/g) |
|---|---|---|
| Example 2-1(A-1) | 2 mm/Extrudate | 77.86 |
| Example 2-2(A-2) | 1.2 mm/Extrudate | 60.32 |
| Example 2-3(A-3) | 2 mm/Extrudate | 129.82 |
| Example 2-4(A-4) | 2 mm/Extrudate | 129.42 |
| Example 2-5(A-5) | 2 mm/Extrudate | 71.87 |
| Example 2-6(A-6) | 2 mm/Extrudate | 131.17 |
| Example 2-7(A-7) | 2 mm/Extrudate | 45.09 |
| Example 2-8(A-8) | 2 mm/Extrudate | 83.71 |
| Comparative Example 2-1(B-1) | 2.4 mm/Tablet | 82.40 |

(2) Hg porosity analysis

The pore structures of the macro and meso regions of the catalysts of Example 2-1 and Comparative Example 2-1 were confirmed through Hg porosity, and the results are shown in Table 5 and FIG. 6.

[Table 5]

| Category | macro pore (mL/g)/(vol%)* | meso pore (mL/g)/(vol%)* | Bulk density (mL/g) | Apparent density (g/mL) |
|---|---|---|---|---|
| Example 2-1(A-1) | 0.0153/2.63 | 0.5662/97.37 | 1.0082 | 3.0764 |
| Example 2-2(A-2) | 0.0153/2.76 | 0.5389/97.24 | 0.8948 | 2.6645 |
| Example 2-3(A-3) | 0.0187/3.04 | 0.5974/96.96 | 0.9159 | 2.7116 |
| Example 2-5(A-5) | 0.0558/8.84 | 0.5753/91.16 | 0.9671 | 3.6060 |
| Example 2-6(A-6) | 0.0575/7.17 | 0.7441/92.83 | 0.7807 | 2.8106 |

(continued)

| Category | macro pore (mL/g)/(vol%)* | meso pore (mL/g)/(vol%)* | Bulk density (mL/g) | Apparent density (g/mL) |
|---|---|---|---|---|
| Comparative Example 2-1(B-1) | 0.0799/17.04 | 0.3889/82.96 | 1.1214 | 3.0060 |
| * It means the ratio of the total mixed volume of the macro pore and the meso pore | | | | |

As can be seen in Tables 4, 5 and FIG. 4, it was confirmed that in order to minimize the decrease in activity due to the diffusion resistance of the internal/external materials, it is desirable to prepare an extrusion molded catalyst through extrudate using an appropriate organic binder and solvent limited in the present disclosure, thereby forming appropriate pores from the meso to micro range, and at the same time having an appropriate range of BET surface area.

By achieving the corresponding pore distribution and BET surface area value, the material diffusion resistance can be minimized during the alkylation reaction, thereby exhibiting high conversion and selectivity, and this will be described in more detail in Experimental Example 2-2.

**[Example 3 and Comparative Example 3: Ortho-alkylation reaction using an ortho alkylation reaction extrusion molded catalyst (B)]**

Example 3-1: Preparation of alkylation reaction product of m-cresol

The alkylation reaction of m-cresol was performed through a continuous flow gas-phase reactor. First, 1g of the molded catalyst of Example 1-1 with the physical properties in Table 1 was charged into a stainless steel tube type reactor with a diameter of 1/2 inch x a length of 50 cm, and then installed in the main furnace.

Then, $N_2$, i.e., a carrier gas, was flowed (37.5 cc/min) using a mass flow controller(MFC), and the inside of the reactor was raised to the activation temperature (450°C) for catalyst activation, and maintained for 1 hour. Then, the monomer composition (12.5 mg/min, m-cresol: Methanol: D.I. water = 1:5:1, LHSV 0.75/h) was vaporized through a preheater (250°C) and then introduced. The reaction composition including the catalyst and the monomer composition was introduced into the reactor, and then the alkylation reaction (450°C, maintained at normal pressure) was performed for 5 hours to obtain a reaction product.

The product during the reaction was collected in a liquid form (IPA (isopropyl alcohol) was used as a solvent) for analysis.

Examples 3-2 to 3-8, Comparative Example 3-1

The catalyst was subjected to an alkylation reaction in the same manner as in Example 3-1, except that the catalyst of Examples 2-2 to 2-8 and Comparative Example 2-1 in Table 3 were used instead of the catalyst of Example 2-1, to thereby obtain the reaction product.

**[Experimental Example 3-2: Analysis of ortho-alkylation reaction product]**

**(3-2-1) Analysis of reaction products according to the type of catalyst**

The liquid in which the reaction products prepared in Example 3 and Comparative Example 3 were collected were analyzed using a gas chromatography(GC) device equipped with a flame ionization detector(FID). From the GC result values, the conversion of m-cresol and the selectivity for target products (2,3,6-TMP, 2,5-DMP, 2,3-DMP, Tetra-methyl phenol) were calculated using the following Mathematical Equations 1 and 2. The results are shown in Table 6, FIG. 4 (type of catalyst (size/shape)) and FIG. 5 (type/content of binder used in catalyst preparation).

The target product was defined as the final product, 2,3,6-TMP, and intermediate materials, 2,5-DMP and 2,3-DMP, from which the final products can be obtained through further reaction.

[Mathematical Equation 1]

$$\text{Conversion of m-cresol (\%)} = \frac{\textit{Mole number of reacted m-cresol}}{\textit{Mole number of fed m-cresol}} \times 100$$

[Mathematical Equation 2]

$$\text{Selectivity for trarget product (\%)} = \frac{\text{Mole number of produced 2,5-DMP + 2,3-DMP + 2,3,6-TMP}}{\text{Mole number of reacted c-cresol}} \times 100$$

[Table 6]

| Category | Type of catalyst | Conversion | Selectivity | | | |
|---|---|---|---|---|---|---|
| | | m-cresol | Target product[a] | DMP[b] | TMP[c] | TeMP[d] |
| Example 3-1 | Example 2-1(A-1) | 96.13 | 88.51 | 21.15 | 62.45 | 8.59 |
| Example 3-2 | Example 2-2(A-2) | 93.58 | 95.39 | 37.91 | 48.87 | 2.2 |
| Example 3-3 | Example 2-3(A-3) | 97.91 | 85.25 | 15.71 | 66.02 | 11.57 |
| Example 3-4 | Example 2-4(A-4) | 97.14 | 87.13 | 19.21 | 63.65 | 9.77 |
| Example 3-5 | Example 2-5(A-5) | 93.50 | 89.94 | 27.2 | 56.57 | 6.98 |
| Example 3-6 | Example 2-6(A-6) | 99.40 | 83.58 | 9.98 | 71.51 | 13.52 |
| Example 3-7 | Example 2-7(A-7) | 93.74 | 91.96 | 29.14 | 56.10 | 5.72 |
| Example 3-8 | Example 2-8(A-8) | 94.95 | 93.99 | 26.47 | 61.76 | 4.69 |
| Comparative Example 3-1 | Comparative Example 2-1(B-1) | 85.01 | 92.68 | 29.16 | 56.0 | 4.82 |

[a] Target product : DMP and TMP
[b] DMP : 2,5-dimethylphenol and 2,3-dimethylphenol
c TMP : 2,3,6-trimethylphenol
d TeMP : Tetra- methyl phenol

As can be seen from the experimental data in Table 6, it was confirmed that when the extrusion molded catalyst according to the present disclosure is used, it exhibits high catalytic activity even by a single catalyst, and when an ortho-alkylation reaction product is prepared using this catalyst, it can exhibit remarkably high selectivity and conversion.

It could be confirmed that in Comparative Example 3-1, it was difficult to achieve a desired degree of catalytic activity by using a tablet-shaped molded catalyst, and accordingly, the conversion, etc. are lowered compared to Examples.

### (3-2-2) Analysis of products according to reaction conditions

Example 4: Change in reaction temperature

The alkylation reaction was performed in the same manner as in Example 3-1, except that the methylation reaction temperature was started from 400°C and changed to 450°C by increasing by 10°C every 3 hours. The conversion of m-cresol and the selectivity for target products (2,3,6-TMP, 2,5-DMP, 2,3-DMP) were calculated according to the method of Experimental Example 3-2 described above, and the results are shown in Table 7 below.

Example 5: Change in carrier gas flow rate

The alkylation reaction was performed in the same manner as in Example 3-1, except that the carrier gas flow rate was 18.7 cc/min, and the conversion of m-cresol and the selectivity for target products (2,3,6-TMP, 2,5-DMP, 2,3-DMP) were calculated according to the method of Experimental Example 3-2 mentioned above, and the results are shown in

Table 7 below.

Example 6: Change in methanol injection amount

The alkylation reaction was performed in the same manner as in Example 3-1, except that m-cresol: Methanol: D.I. water = 1:4:1 (11.1 mg/min, LHSV 0.666/h) and m-cresol: Methanol: D.I. water = 1:3:1 (9.7 mg/min, LHSV 0.582/h) were injected as the liquid mixture including the reactants in Example 3-1, and the conversion of m-cresol and the selectivity for target products (2,3,6-TMP, 2,5-DMP, 2,3-DMP) were calculated according to the method in Experimental Example 3-2 mentioned above, and the results are shown in Table 7 below.

[Table 7]

| Category (type of catalyst) | Reaction condition | Conversion | Selectivity | | | |
|---|---|---|---|---|---|---|
| | | m-cresol | Target product[a] | DMP[b] | TMP[c] | TeMP[d] |
| Example 4 (A-1) | 400 °C | 47.12 | 97.16 | 82.32 | 14.84 | 0 |
| | 410 °C | 60.58 | 97.22 | 76.26 | 20.96 | 0.49 |
| Adjustment of reaction temperature | 420 °C | 70.09 | 96.97 | 67.5 | 29.47 | 0.98 |
| | 430 °C | 84.91 | 95.28 | 46.16 | 49.12 | 2.93 |
| | 440 °C | 90.38 | 94.12 | 36.22 | 57.9 | 4.06 |
| | 450 °C | 95.55 | 92.41 | 24.74 | 67.67 | 5.75 |
| Example 5 | 37.5 cc/min | 96.13 | 88.51 | 21.15 | 62.45 | 8.59 |
| (A-1) | (the same as Example 3-1) | | | | | |
| Adjustment of nitrogen flow rate | 18.7 cc/min | 98.76 | 87.67 | 12.65 | 75.02 | 9.93 |
| Example 6 (A-1) | 1:3:1 | 59.98 | 96.14 | 75.45 | 20.69 | 0.56 |
| | 1:4:1 | 96.29 | 89.61 | 26.95 | 62.66 | 7.54 |
| Adjustment of feed ratio | 1:5:1 (the same as Example 3-1) | 96.13 | 88.51 | 26.06 | 62.45 | 8.59 |

[a] Target product : DMP and TMP
[b] DMP : 2,5-dimethylphenol and 2,3-dimethylphenol
c TMP : 2,3,6-trimethylphenol
d TeMP : Tetra- methyl phenol

As can be seen from the experimental data in Table 7, it could be confirmed that the methylation reaction temperature was started from 400°C and changed to 450°C by increasing by 10°C every 3 hours, though which the catalytic performance increases depending on the reaction temperature.

It could be confirmed that Example 5, in which the amount of nitrogen input was changed, has the effect on the contact time between the catalyst and the reactant (GHSV, Gas hourly space velocity).

It could be confirmed that Example 6, in which the ratio of the feed introduced was changed, has the effect of the ratio of meta-cresol, the reactant, and methanol as the alkylation medium.

## Claims

**1.** An ortho-alkylation reaction catalyst comprising:
magnesium oxide having a bimodal pore structure and a BET surface area of 100 $m^2$/g to 180 $m^2$/g.

**2.** The ortho-alkylation reaction catalyst according to claim 1, wherein, in the bimodal pore structure,

the diameter of a first pore is 2 nm to 10 nm, and

the diameter of a second pore is 10 nm to 50 nm.

**3.** The ortho-alkylation reaction catalyst according to claim 1, wherein: the BET surface area is 130 $m^2/g$ to 180 $m^2/g$.

**4.** An ortho-alkylation reaction extrusion molded catalyst,

comprising magnesium oxide, and
comprising a macro pore having a diameter of 50 nm to 10,000 nm and a meso pore having a diameter of 2 nm to 50 nm,
wherein a BET surface area is 45 $m^2/g$ to 180 $m^2/g$.

**5.** The ortho-alkylation reaction extrusion molded catalyst according to claim 1, wherein:

based on the total mixed volume of the macro pore and the meso pore,
the macro pore is included in an amount of 1 to 10 vol%, and
the meso pore is included in an amount of 90 to 99 vol%.

**6.** The ortho-alkylation reaction extrusion molded catalyst according to claim 1, wherein: the size of the extrusion molded catalyst is 0.5 mm to 6.0 mm.

**7.** A method for preparing the catalyst as set forth in claim 4, comprising:

a step 1 of preparing a mixture of magnesium oxide having a bimodal pore structure and a BET surface area of 100 $m^2/g$ to 180 $m^2/g$, an organic binder and a solvent; and
a step 2 of extrusion-molding the mixture.

**8.** The method for preparing the catalyst according to claim 7, wherein the bimodal pore structure comprises,

a first pore having a diameter of 2 nm to 10 nm, and
a second pore having a diameter of 10 nm to 50 nm.

**9.** The method for preparing the catalyst according to claim 7, wherein:

based on 100 parts by weight of the magnesium oxide,
the organic binder resin is included in an amount of 0.1 to 20 parts by weight, and
the solvent is included in an amount of 50 to 200 parts by weight.

**10.** The method for preparing the catalyst according to claim 7, wherein: the organic binder comprises at least one selected from the group consisting of methyl cellulose, carboxymethyl cellulose, ethylene glycol, polyethylene glycol, polyphenylene oxide, glycerin and propylene glycol.

**11.** The method for preparing the catalyst according to claim 7, wherein: the solvent is alcohol, water, or a mixture thereof.

**12.** The method for preparing the catalyst according to claim 7, wherein: the solvent is water.

**13.** The method for preparing the catalyst according to claim 7, wherein: the extrusion molding of the step 2 is performed by using a single piston extrusion molding machine.

**14.** The method for preparing the catalyst according to claim 7, further comprising drying and baking after the extrusion molding of the step 2.

**15.** The method for preparing the catalyst according to claim 14, wherein: the drying is performed at 80°C to 120°C.

**16.** The method for preparing the catalyst according to claim 14, wherein:

the baking is performed at 300°C to 600°C.

**17.** An ortho-alkylation reaction composition comprising: the ortho-alkylation reaction catalyst as set forth in claim 1 or the ortho-alkylation reaction extrusion molded catalyst as set forth in claim 4, and a monomer composition comprising a meta-alkyl substituted phenolic monomer.

**18.** The ortho-alkylation reaction composition according to claim 17, wherein:
the meta-alkyl substituted phenolic monomer is meta-cresol.

**19.** The ortho-alkylation reaction composition according to claim 17, wherein:
the monomer composition further comprises an alkanol and distilled water.

**20.** The ortho-alkylation reaction composition according to claim 19, wherein:
the monomer composition comprises phenolic monomer: alkanol: distilled water in a ratio of 1:3 to 10:1 to 5 parts by weight.

**21.** A preparation method of ortho-alkylation reaction product, comprising:
performing an alkylation reaction of a monomer composition containing a meta-alkyl substituted phenolic monomer in the presence of the ortho-alkylation reaction catalyst as set forth in claim 1 or the ortho-alkylation reaction extrusion molded catalyst as set forth in claim 4.

**22.** The preparation method of ortho-alkylation reaction product according to claim 21, wherein:
the alkylation reaction is performed at 350 to 550°C.

**23.** The preparation method of ortho-alkylation reaction product according to claim 21, wherein:
the alkylation reaction is performed by using a continuous flow gas-phase reactor.

**24.** The preparation method of ortho-alkylation reaction product according to claim 23, wherein:
the monomer composition is injected into a continuous flow gas-phase reactor at a liquid hourly space velocity(LHSV) of 0.5 $hr^{-1}$ to 2.0 $hr^{-1}$.

**25.** The preparation method of ortho-alkylation reaction product according to claim 21, wherein:
the ortho-alkylation reaction product is at least one selected from the group consisting of 2,5-dimethylphenol, 2,3-dimethylphenol, 2,3,6-trimethylphenol, 3-methylanisole, 3,4-dimethylphenol and tetramethylphenol.

*FIG. 1*

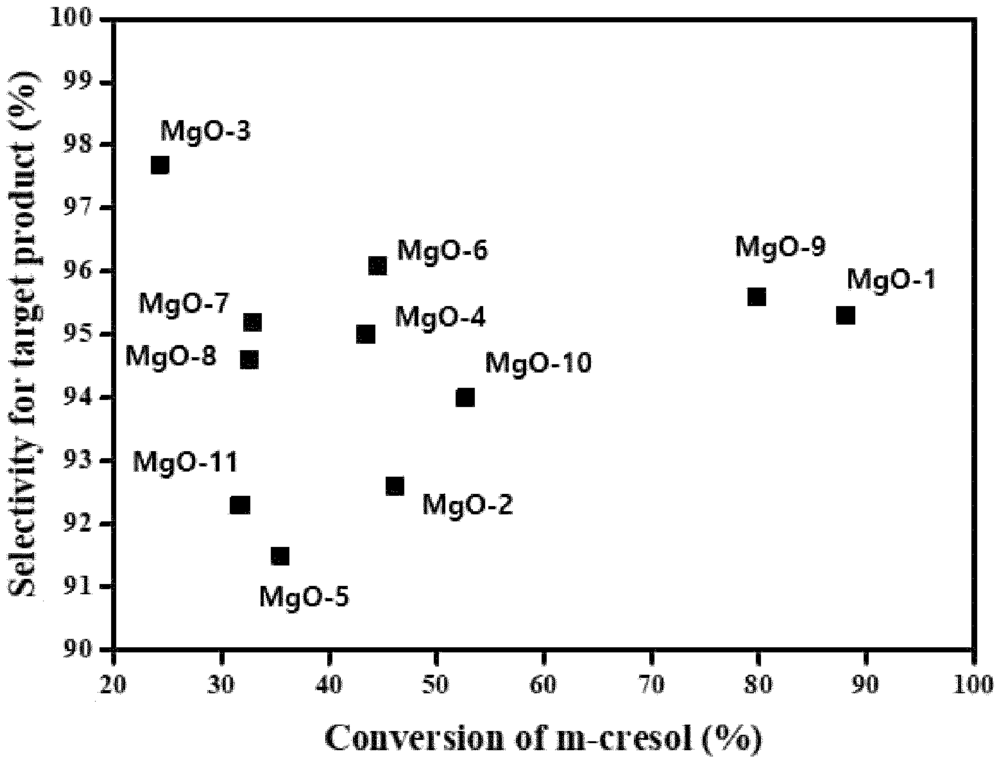
MgO-3
MgO-6
MgO-9
MgO-1
MgO-7
MgO-4
MgO-8
MgO-10
MgO-11
MgO-2
MgO-5
Selectivity for target product (%)
Conversion of m-cresol (%)
100
99
98
97
96
95
94
93
92
91
90
20
30
40
50
60
70
80
90
100

*FIG. 2*

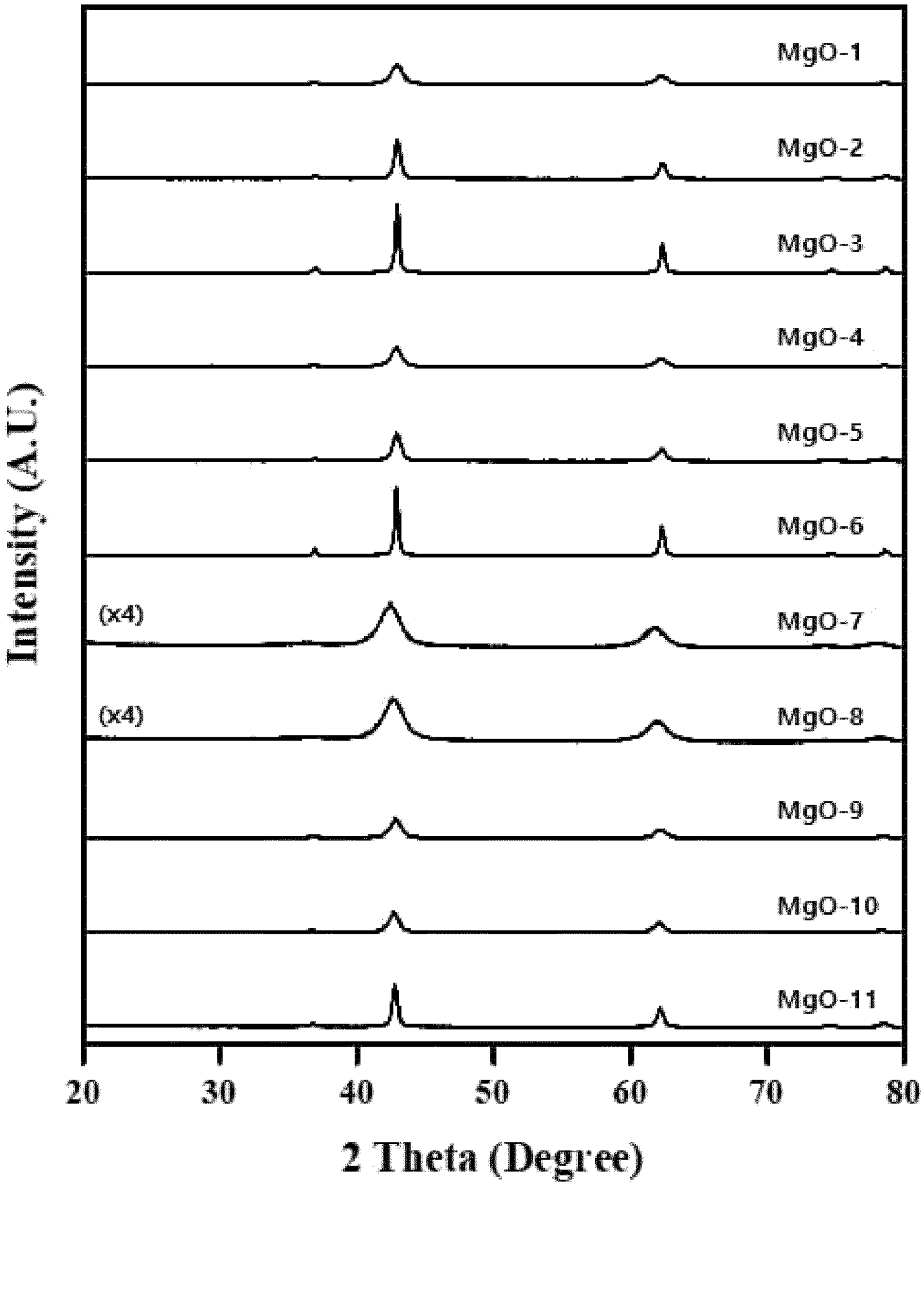
MgO-1
MgO-2
MgO-3
MgO-4
MgO-5
MgO-6
(x4)
MgO-7
(x4)
MgO-8
MgO-9
MgO-10
MgO-11
Intensity (A.U.)
20
30
40
50
60
70
80
2 Theta (Degree)

*FIG. 3*

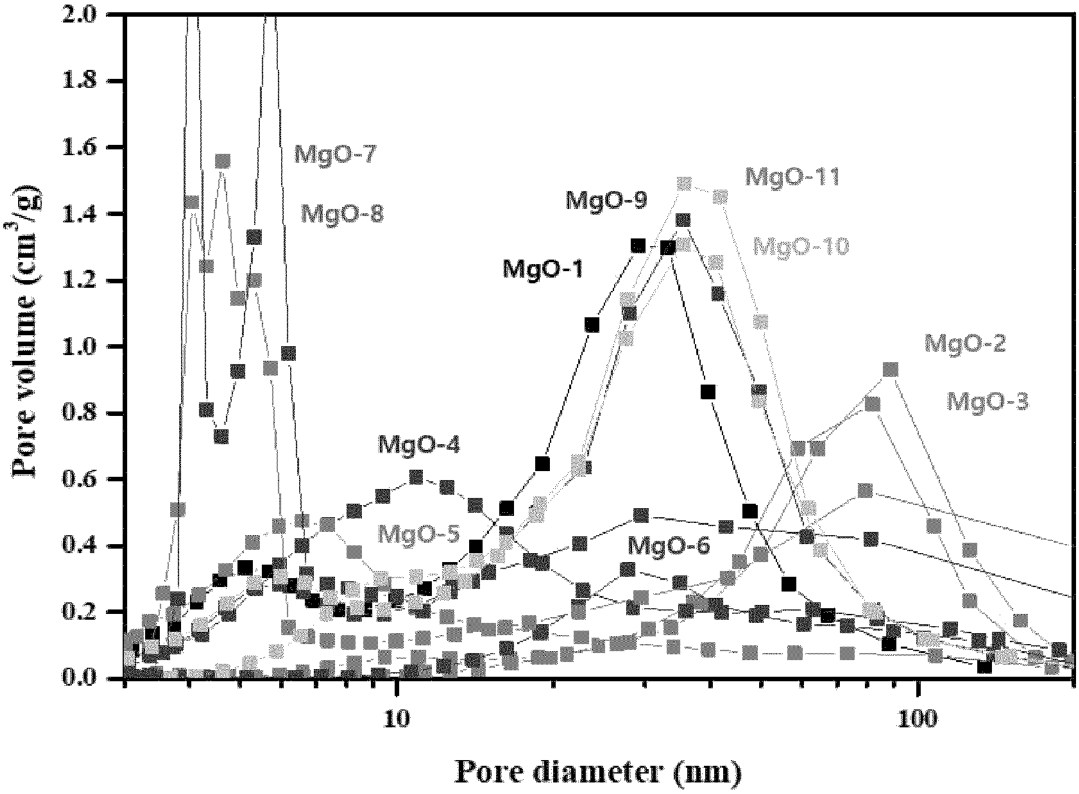
MgO-7
MgO-8
MgO-9
MgO-1
MgO-11
MgO-10
MgO-2
MgO-3
MgO-4
MgO-5
MgO-6
Pore volume (cm³/g)
Pore diameter (nm)
2.0
1.8
1.6
1.4
1.2
1.0
0.8
0.6
0.4
0.2
0.0
10
100

*FIG. 4*

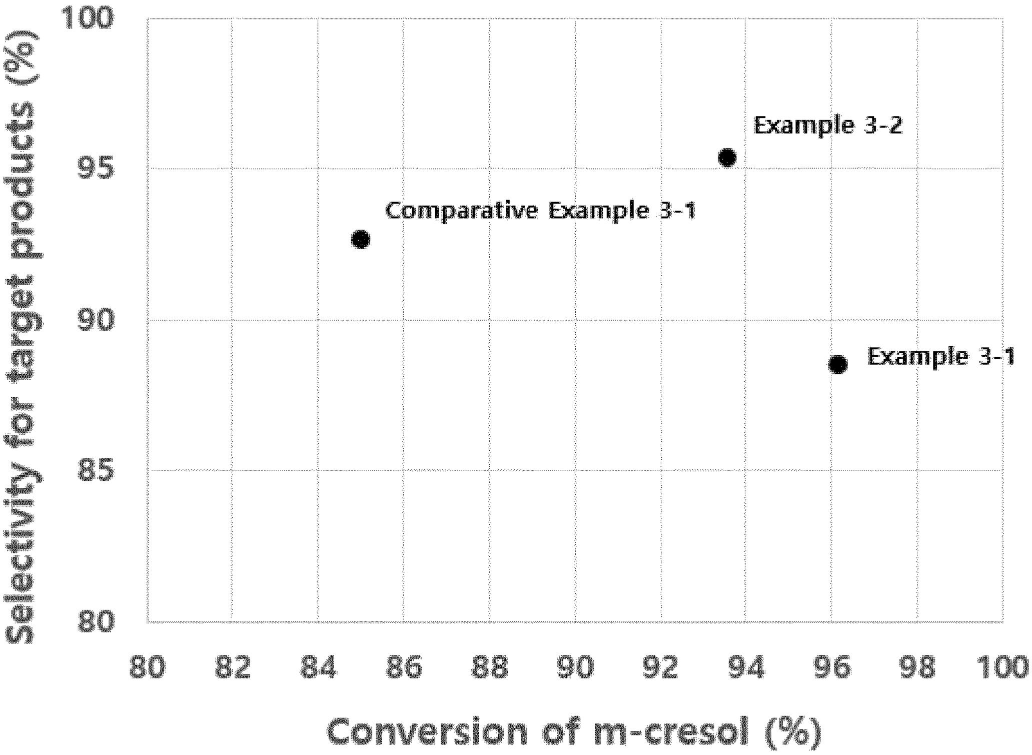
Selectivity for target products (%)
100
95
90
85
80
Example 3-2
Comparative Example 3-1
Example 3-1
80
82
84
86
88
90
92
94
96
98
100
Conversion of m-cresol (%)

*FIG. 5*

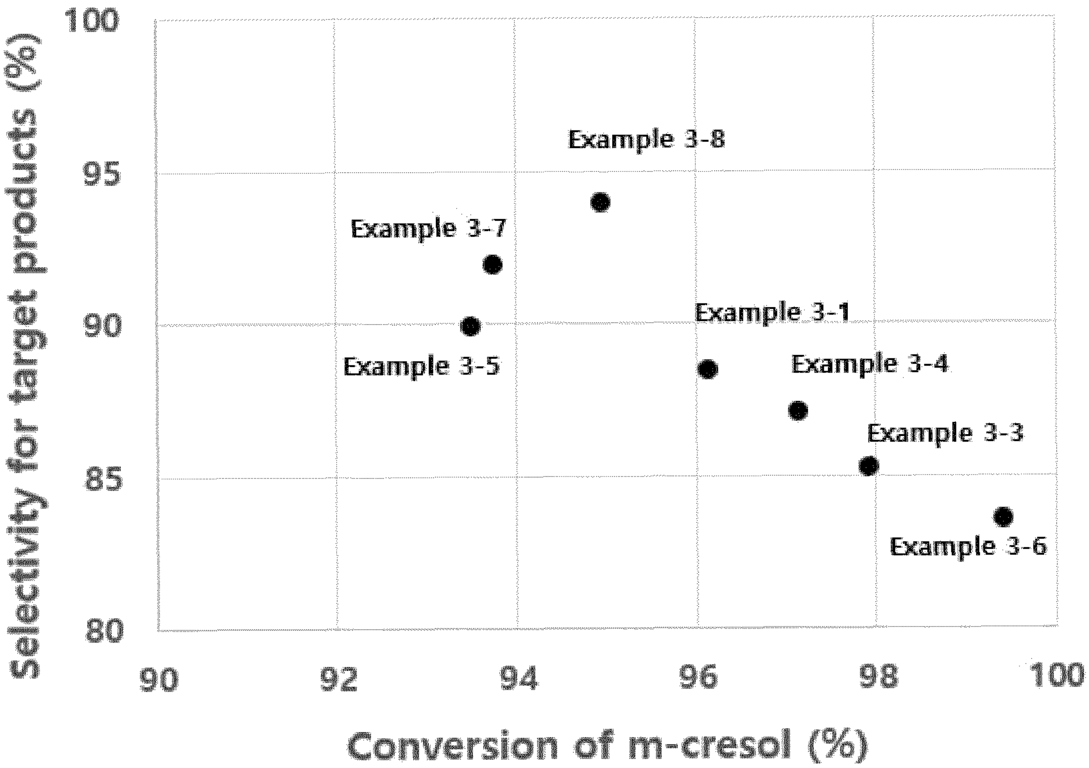
Selectivity for target products (%)
100
95
90
85
80
90
92
94
96
98
100
Conversion of m-cresol (%)
Example 3-8
Example 3-7
Example 3-5
Example 3-1
Example 3-4
Example 3-3
Example 3-6

*FIG. 6*

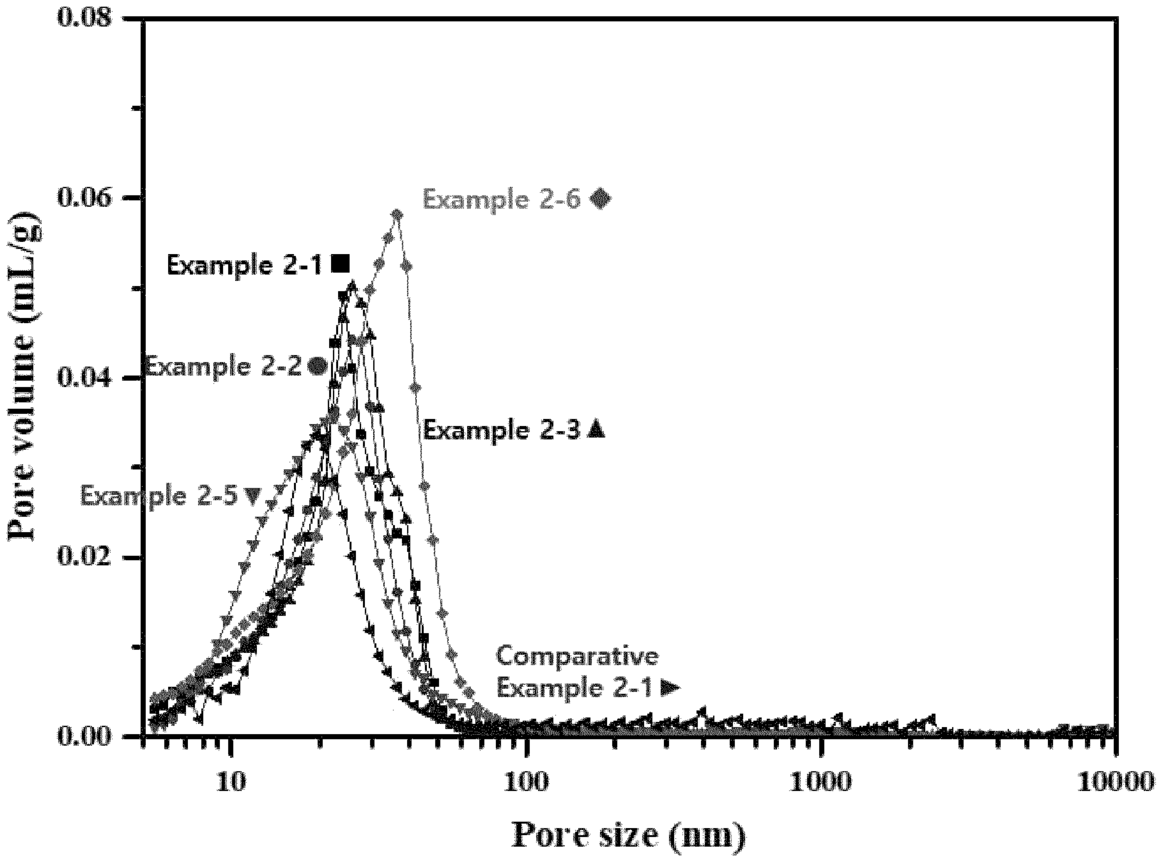
0.08
0.06
0.04
0.02
0.00
Pore volume (mL/g)
Example 2-6
Example 2-1
Example 2-2
Example 2-3
Example 2-5
Comparative
Example 2-1
10
100
1000
10000
Pore size (nm)

**INTERNATIONAL SEARCH REPORT**

International application No. **PCT/KR2023/006330**

**A. CLASSIFICATION OF SUBJECT MATTER**

**B01J 23/02**(2006.01)i; **B01J 35/10**(2006.01)i; **B01J 37/00**(2006.01)i; **C07C 37/11**(2006.01)i; **C07C 39/06**(2006.01)i; **B01J 21/10**(2006.01)i

According to International Patent Classification (IPC) or to both national classification and IPC

**B. FIELDS SEARCHED**

Minimum documentation searched (classification system followed by classification symbols)

B01J 23/02(2006.01); B01J 21/10(2006.01); B01J 23/755(2006.01); B01J 23/78(2006.01); B01J 23/847(2006.01); B01J 23/889(2006.01); B01J 35/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 오르토-알킬화(ortho-alkylation), 촉매(catalyst), 산화마그네슘(magnesium oxide), 바이모달(bimodal), 매크로 기공(macropore), 메조 기공(mesopore), 압출(extrusion)

**C. DOCUMENTS CONSIDERED TO BE RELEVANT**

| Category* | Citation of document, with indication, where appropriate, of the relevant passages | Relevant to claim No. |
|---|---|---|
| X<br>Y | KR 10-2004-0045497 A (GENERAL ELECTRIC COMPANY) 01 June 2004 (2004-06-01)<br>See claims 18-21; and paragraphs [0003]-[0050]. | 1-3,17,19-24<br>4-16,18,25 |
| Y | KR 10-2013-0066916 A (KOREA INSTITUTE OF ENERGY RESEARCH) 21 June 2013 (2013-06-21)<br>See claims 1-4; and paragraphs [0001]-[0061]. | 4-16 |
| Y | CROCELLÀ, V. et al. The balance of acid, basic and redox sites in Mg/Me-mixed oxides: The effect on catalytic performance in the gas-phase alkylation of m-cresol with methanol. Journal of Catalysis. 2010, vol. 270, no. 1, pp. 125-135.<br>See abstract. | 18,25 |
| A | KR 10-2011-0107628 A (KOREA RESEARCH INSTITUTE OF CHEMICAL TECHNOLOGY) 04 October 2011 (2011-10-04)<br>See entire document. | 1-25 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 August 2023** | **21 August 2023** |

Name and mailing address of the ISA/KR

**Korean Intellectual Property Office**
**Government Complex-Daejeon Building 4, 189 Cheongsa-ro, Seo-gu, Daejeon 35208**

Facsimile No. **+82-42-481-8578**

Authorized officer

Telephone No.

Form PCT/ISA/210 (second sheet) (July 2022)

**INTERNATIONAL SEARCH REPORT**

International application No.
**PCT/KR2023/006330**

**C. DOCUMENTS CONSIDERED TO BE RELEVANT**

| Category* | Citation of document, with indication, where appropriate, of the relevant passages | Relevant to claim No. |
|---|---|---|
| A | WO 2021-134013 A1 (SHPP GLOBAL TECHNOLOGIES B.V. et al.) 01 July 2021 (2021-07-01)<br>See entire document. | 1-25 |

Form PCT/ISA/210 (second sheet) (July 2022)

**INTERNATIONAL SEARCH REPORT**
**Information on patent family members**

International application No.
**PCT/KR2023/006330**

| Patent document cited in search report | Publication date (day/month/year) | Patent family member(s) | Publication date (day/month/year) |
|---|---|---|---|
| KR 10-2004-0045497 A | 01 June 2004 | CN 1633335 A | 29 June 2005 |
| | | CN 1633335 B | 16 June 2010 |
| | | EP 1438131 A1 | 21 July 2004 |
| | | JP 2005-505407 A | 24 February 2005 |
| | | KR 10-0720219 B1 | 21 May 2007 |
| | | US 2003-0073572 A1 | 17 April 2003 |
| | | US 2005-0004407 A1 | 06 January 2005 |
| | | US 6897175 B2 | 24 May 2005 |
| | | US 7208438 B2 | 24 April 2007 |
| | | WO 03-031057 A1 | 17 April 2003 |
| KR 10-2013-0066916 A | 21 June 2013 | KR 10-1394287 B1 | 13 May 2014 |
| KR 10-2011-0107628 A | 04 October 2011 | KR 10-1070169 B1 | 05 October 2011 |
| WO 2021-134013 A1 | 01 July 2021 | CN 114514069 A | 17 May 2022 |
| | | EP 3842138 A1 | 30 June 2021 |
| | | EP 4081341 A1 | 02 November 2022 |
| | | JP 2023-508385 A | 02 March 2023 |
| | | US 2023-0048457 A1 | 16 February 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020220058310
- KR 1020230055405